# EUROPEAN PATENT SPECIFICATION

(11) **EP 2 127 669 B1**
(45) Date of publication and mention of the grant of the patent: **14.08.2013**
(21) Application number: 08736676.1
(22) Date of filing: 11.02.2008
(51) Int. Cl.: A61K 38/48, A61P 7/02

(54) **USE OF MATRIX METALLOPROTEINASE-10 (MMP-10) FOR THROMBOLYTIC TREATMENTS**
VERWENDUNG VON MATRIX-METALLOPROTEINASE-10 (MMP-10) FÜR THROMBOLYSEBEHANDLUNGEN
UTILISATION DE LA MÉTALLOPROTÉINASE MATRICIELLE-10 (MMP-10) DANS DES TRAITEMENTS THROMBOLYTIQUES

(30) Priority: 26.02.2007 ES 200700501
(43) Date of publication of application: 02.12.2009
(73) Proprietor: Proyecto De Biomedicina Cima, S.L., 31180 Zizur Mayor (Navarra) (ES)
(72) Inventor: ORBE LOPATEGUI, Josune, 31008 Pamplona (Navarra) (ES); RODRÍGUEZ GARCÍA, José Antonio, 31008 Pamplona (Navarra) (ES); PÁRAMO FERNÁNDEZ, José Antonio, 31008 Pamplona (Navarra) (ES); SERRANO VARGAS, Rosario, 31008 Pamplona (Navarra) (ES)
(74) Representative: Ungria López, Javier
(86) International application number: PCT/ES2008/000072
(87) International publication number: WO 2008/104620

(56) References cited:
- EP-A2- 1 060 747
- EP-A2- 1 060 747
- WO-A2-03/028756
- LIJNEN H R ET AL: "Plasminogen/plasmin and matrix metalloproteinase system function in mice with targeted inactivation of stromelysin-3 (MMP-11)", FIBRONOLYSIS. INTERNATIONAL JOURNAL OF FIBRINOLYSIS,THROMBOLYSIS AND ECTRACELLULAR PROTEOLYSIS, XX, XX, vol. 12, no. 3, 1 May 1998 (1998-05-01), pages 155-164, XP004921097, ISSN: 0268-9499, DOI: 10.1016/S0268-9499(98)80297-X
- ORBE J ET AL: "Independent association of matrix metalloproteinase-10, cardiovascular risk factors and subclinical atherosclerosis", JOURNAL OF THROMBOSIS AND HAEMOSTASIS, BLACKWELL PUBLISHING, OXFORD, GB, vol. 5, no. 1, 1 January 2007 (2007-01-01) , pages 91-97, XP008112472, ISSN: 1538-7933, DOI: 10.1111/J.1538-7836.2006.02276.X [retrieved on 2006-10-16]
- LIJNEN H R ET AL: "Plasminogen/plasmin system function in mice deficient in stromelysin-1 (MMP-3) or in tissue inhibitor of metalloproteinases type-1 (TIMP-1)", FIBRONOLYSIS. INTERNATIONAL JOURNAL OF FIBRINOLYSIS,THROMBOLYSIS AND ECTRACELLULAR PROTEOLYSIS, XX, XX, vol. 12, no. 1, 1 January 1998 (1998-01-01), pages 1-8, XP004920006, ISSN: 0268-9499, DOI: 10.1016/S0268-9499(98)80002-7
- SAUNDERS W B ET AL: "MMP-1 Activation By Serine Protease and MMP-10 Induces Human Capillary Tubular Network Collapse and Regression in 3D Collegen Matrices", JOURNAL OF CELL SCIENCE, CAMBRIDGE UNIVERSITY PRESS, LONDON, GB, vol. 118, 1 January 2005 (2005-01-01), pages 2325-2340, XP003007483, ISSN: 0021-9533, DOI: 10.1242/JCS.02360
- HIRAOKA N ET AL: "Matrix metalloproteinases regulate neovascularization by acting as pericellular fibrinolysins", CELL, CELL PRESS, US, vol. 95, 30 October 1998 (1998-10-30), pages 365-377, XP002179644, ISSN: 0092-8674, DOI: 10.1016/S0092-8674(00)81768-7
- PARAMO J A ET AL: "Association Between Matrix Metalloproteinase-10 Concentration and Smoking in Individuals Without Cardiovascular Disease", REVISTA ESPANOLA DE CARDIOLOGIA, EDICIONES DOYMA S.A., BARCELONA, ES, vol. 61, no. 12, 1 January 2008 (2008-01-01), pages 1267-1273, XP026028450, ISSN: 1885-5857, DOI: 10.1016/S1885-5857(09)60054-2 [retrieved on 2008-01-01]
- RODRÍGUEZ J A ET AL: "Metalloproteases,Vascular Remodeling, and Atherothrombotic Syndromes", REVISTA ESPANOLA DE CARDIOLOGIA, MADRID, ES, vol. 60, no. 9, 1 September 2007 (2007-09-01), pages 959-967, XP002631753, ISSN: 0300-8932
- HIRAOKA N. ET AL.: 'Matrix metalloproteinase regulate neovascularization by acting as pericellular fibrinolysins' CELL vol. 95, 30 October 1998, pages 365 - 377, XP002179644
- ORBE J. ET AL.: 'Independent association of matrix metalloproteinase-10, cardiovascular risk factors and subclinical atherosclerosis' JOURNAL OF THROMBOSIS AND HAEMOSTASIS vol. 5, January 2007, pages 91 - 97, XP008112472

## Description

### FIELD OF THE INVENTION

This invention relates to pharmaceutical compositions which include matrix metalloproteinase-10 (MMP10) and, more specifically, a combination of MMP-10 and a plasminogen activator, and the use thereof for thrombolytic therapy and treatment.

### STATE-OF-THE-ART PRIOR TO THE INVENTION

The haemostatic system is the system in charge of maintaining circulatory flow and preventing hemorrhage in response to vascular attack. Physiological hemostasis is controlled both by the mechanisms which promote coagulation and fibrin formation, as well as those which favor the degradation thereof or fibrinolysis. Excessive activation of coagulation or a defect in fibrinolysis gives rise to the formation of clots which block the blood vessels (intravascular thrombosis), causing ischemia and necrosis. However, an overall situation of hyperfibrinolysis will favor the onset of hemorrhages.

Cardiovascular diseases of an atherothrombotic nature are today the main cause of morbid-mortality. Within this group of diseases, thrombotic processes are the main mechanism giving rise to acute cardiovascular events of major clinical importance, such as acute myocardial infarction (MI) or cerebrovascular accident (stroke).

Therefore, all of the strategies for treating cerebrovascular accidents and thrombotic events in general must necessarily promote the rapid rerouting of the arterial passage blocked by the clot in order to restore blood flow to the tissues and thus prevent any greater damage. This is what is commonly known as thrombolytic therapy.

Given that fibrinolysis is the underlying biochemical process of thrombolysis, thrombolytic therapy seeks, first of all, to favor the degradation of the fibrin network which is holding the clot together.

Given that plasmin is the enzyme which catalyzes the lysis and degradation of fibrin, the first objective for achieving a rapid dissolving of the clot is to maximize the generation of plasmin.

For this purpose, the use of plasminogen activators, capable of activating the conversion of the plasminogen (inactive proenzyme) into active plasmin: tissue plasminogen activator (tPA), urokinase plasminogen activator (uPA) or other similar agents was introduced as of 1980.

Baker [Clin. Appl. Thrombosis/Hemostasis, 2002; 8:291-314] conducts a review of the state-of-the-art in thrombolytic therapy and of the thrombolytic agents in use or in development, the clinical application thereof, as well as advantages and drawbacks. In this document, Baker also sets out the characteristics which an ideal thrombolytic agent should have: 1) fast-acting thrombolysis, for the rapid restoring of arterial or venous flow; 2) fibrin specificity, so that the fibrinolysis will be confined to the areas of acute thrombosis with a reduced systemic fibrinolysis; 3) sustained action over time; 4) clot specificity so as to prevent effects on the fibrinogen, other proteins involved in coagulation and not to alter primary hemostasis; 5) no side-effects; and 6) low cost.

In the case of acute myocardial infarction and of acute cerebrovascular ischemia (stroke), the success of the thrombolytic treatment leads to an increase in the survival of the patients and a better recovery of the function of the ischemic tissue [White HD et al.; N. Engl. J. Med., 1987; 317:850-855]; [Suwanwela N and Koroshetz WJ; Annu. Rev. Med., 2007; 58:89-106]. Unfortunately, fibrinolytic treatment has failures and side-effects.

Almost 40% of the patients with acute myocardial infarction do not respond to fibrinolytic treatment and do not achieve an optimum rerouting of the artery blocked by the thrombus [Armstrong PW and Collen D; Circulation, 2001; 103:2862-2866].

To solve this problem, instead of pharmacological thrombolysis, primary percutaneous angioplasty is currently being used as reperfusion treatment more effective than thrombolytic treatment in terms of reducing the death rate, reinfarction and hemorrhage. It is not however possible to use angioplasty in many cases (no haemodynamics laboratory available or geographical distance can not be assumed) and it is then when thrombolytic treatment is performed. Therefore, it is desirable that new treatment strategies be developed which make it possible to improve the effectiveness of the thrombolytic treatment, for example, prehospital fibrinolytic treatment, new thrombolytic agents (tenecteplase), or new pharmacological combinations (i.e., reduce the fibrinolytic agents to half the dose and add a GP IIb/IIIa platelet receptor blocker [Brouwer MA et al., Heart, 2004; 90:581-588]. A considerable death rate also exists related to fibrinolytic treatment due to hemorrhagic complications; specially hemorrhage of the central nervous system and major hemorrhages, with a 2%-14% incidence.

In the case of acute cerebrovascular ischemia, the thrombolytic treatment with recombinant tPA within the first three hours following the onset of the symptoms is the only scheme which has shown itself to be somewhat effective. Unfortunately, in 25%-30% of the cases, the treatment fails, the clot does not lyse, and the blocked artery does not become permeable. Additionally, the treatment with tPA has a high percentage of hemorrhagic complications (up to 5% entail symptomatic hemorrhage), and many physicians fear this complication. For that reason, a large majority of patients who could benefit from this treatment does not receive it. Another problem related to administering tPA, potentially more serious than the risk of hemorrhaging, is the toxicity on the central nervous system which is many times responsible for the therapeutic failure [Cheng T. et al., Nat. Med., 2006; 12:1278-1285]. Therefore, reducing the risk of hemorrhaging from administering tPA could change the perception of the safety of this drug and increase its use. Therefore, it still continues to be necessary to select therapeutic agents and combinations which will make it possible to reduce the toxicity of the tPA either directly or indirectly, by lowering the dose necessary for treating stroke.

Given that there are enzymes different from plasmin which can directly degrade fibrinogen and fibrin, research is also being done on their potential use for thrombolytic treatment. These enzymes include proteases endogenous of leukocytes (elastase and cathepsin G), snake venom or leech proteases or proteases from some bacteria.

In EP1060747, the use of fibrinolytic matrix metalloproteinases is described which show a significant activity for proteolytically cleaving and degrading fibrin and fibrinogen. These fibrinolytic metalloproteinases include MMP-2 (gelatinase A), MMP-3 (stromelysin 1), MMP-7 (matrilysin) MMP-9 and very particularly membrane-type matrix metalloproteinase MMP-MT1. Months later, Bini et al. compiled and expanded upon these same findings [Biochemistry, 1999; 38: 13928-13936]. However, neither in these nor in other later works are data provided concerning the effectiveness of these fibrinolytic are matrix metalloproteinases in the lysis and degradation of the fibrin which forms thrombi, either for achieving a more rapid dissolving of the clot, or rather for providing a greater selectivity for the degradation of the fibrin in the clot respecting the systemic fibrinogen.

The objective of the present invention is to provide alternative therapeutic compositions and combinations for thrombolytic treatment which will favor the lysis of the clots by means of a selective degradation of the fibrin and which will aid in minimizing the adverse effects related to other thrombolytic treatments (hemorrhage, toxicity, etc.).

### BRIEF DESCRIPTION OF THE FIGURES

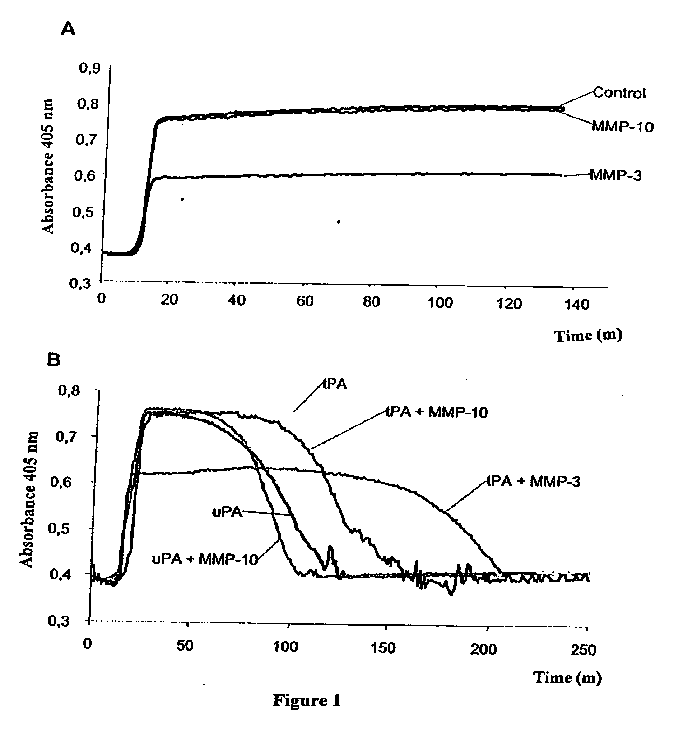

**Figure 1****.** Recalcified plasma turbidimetry test given as the absorbance values at 405 nm compared to the time the experiment lasts in minutes. **A:** the graph shows the differences in clot formation (maximum absorbance) of the plasma alone (control) or in the presence of MMP-10 (200 nM) or MMP-3 (200 nM). **B:** The graph depicts the formation and lysis of the fibrin clot of the recalcified plasma in the presence of tPA plasminogen activators (30 U /ml) and uPA (135 U/ml) alone or combined with MMP-10 (200 nM) and also in the presence of an equivalent dose of MMP-3 (200 nM) combined with tPA (30 U/ml).
**Figure 2**. Polymerized fibrin plate showing the lysis areas caused by the tPA (1 U/ ml) and the MMP-10 (200 nM) individually, or added in combination with one another.
**Figure 3**. Turbidimetry test showing the difference in the lysis time of the fibrin clot at different doses of tPA (20. 25 and 30 U/ml). The combination of MMP-10 (200 nM) and tPA (20 U/ml) shortens the lysis time as compared to the activator by itself.
**Figure 4****.** Test of MMP-10 (100 nM) plasma activity with a fluorescent stromelysin substrate. The monoclonal antibody (MAb) concentration which inhibits the MMP-10 plasma activity was determined by means of the reduction in the slope of the substrate formation. As a control, an IgG isotype control antibody was used.
**Figure 5**. Turbidimetry test of the plasma recalcified with MMP-10 (200 nM) in the presence or absence of a monoclonal antibody (MAb) which inhibits the activity of the MMP-10, and of an isotype (IgG) control antibody.
**Figure 6****.** Fibrin plate illustrating the differences in the lysis area
caused by the tPA (1 U/ml) and the MMP-10 (200 nM) in the presence or absence of a monoclonal antibody which inhibits the activity of the MMP-10 (MAb) and the isotype control antibody (IgG).

### DETAILED DESCRIPTION OF THE INVENTION

This invention relates, firstly, to the use or utilization of matrix metalloproteinase-10 (MMP-10) in the preparation of a medicament for thrombolytic therapy and treatment.

MMP-10, or stromelysin-2, is located in chromosome 11 and is expressed by different cell types, such as the endothelial cells, monocytes and fibroblasts [Madlener M and Werner S; Gene, 1997; 202:75-81]. It is known that it can be activated by plasmin, kallikrein, tryptase, elastase and cathepsin G and can degrade a wide range of substrates of the extracellular matrix, such as aggrecan, elastin, fibronectin, gelatin, laminin, tenascin-C, vitronectin and collagens type II, III, IV, IX, X and XI. Additionally, MMP-10 can activate other matrix metalloproteinases, such as proMMP-1, -3, -7, -8 and -9 [Nakamura H et al., Eur. J. Biochem., 1998; 253: 67-75].

It is likewise known that MMP-10 is involved in different physiological processes, such as bone growth or wound healing. It is also found overexpressed in corneas of patients with diabetic retinopathy and has been related to some types of carcinoma, as well as lymphoid tumors. Different *in vitro* studies have shown that, in keratinocyte cultures, the expression of MMP-10 can be induced both by growth factors (epidermal growth factor of keratinocytes or TGF-beta), as well as by proinflammatory cytokines (TNF-alfa, IL-1beta) [Rechardt O et al., J. Invest. Dermatol., 2000; 115:778-787]; [Li de Q et al.; Invest. Ophthalmol. Vis. Sci. 2003; 44 :2928-2936].

Also in publications prior to this invention, it has been described that MMP-10:
- may be an inflammatory biomarker of vascular risk [Montero I et al.; J. Am. Coll. Cardiol., 2006; 47:1369-1378] [Orbe J et al.; J. Thromb. Haemost.; 2007; 5: 91-97];
- is induced in endothelial cells which are forming capillaries in 3D collagen matrixes and is involved in the regression of the formation of capillaries by means of the activation of MMP-1 [Saunders WB et al.; J. Cell. Sci., 2005; 118: 2325-2340]; and that
- it plays a fundamental role in maintaining the intracellular bonds which preserve vascular integrity in angiogenesis and remodeling processes [Chang S et al.; Cell, 2006; 126:321-334].

In the present invention, the effect of MMP-10 and of MMP-3 on the formation and lysis of clots in human plasma, as well as on other *in vitro* models of polymerized fibrin degradation has been tested.

The inventors have been able to prove that MMP-10 does not have any direct thrombolytic activity and that it is not capable of degrading fibrinogen or fibrin by itself. Surprisingly, they have also proven that, in the presence of thrombolysis-activating agents, particularly plasminogen activators, MMP-10 favors the dissolving of the fibrin clots and shortens lysis time. MMP-10 therefore acts as a facilitator or adjuvant of the thrombolytic action of other thrombolysis activators.

On the contrary, a fibrinolytic matrix metalloproteinase such as MMP-3, which has a direct proteolytic activity on fibrin and fibrinogen, does not shorten the clot lysis times which is provided by the thrombolysis activators by themselves.

Within the context of this invention, "thrombolytic therapy" is understood as being that therapy which, in clinical situations of ischemia of thrombotic origins, the reperfusion or restoring of blood flow by means of the lysis or rapid dissolving of the clots which are blocking the circulation and jeopardizing organ function, is being sought. These clinical situations include, in particular, thrombolytic therapy in acute myocardial infarction, in cerebral thrombosis (more particularly acute cerebral infarction or stroke), as well as other venous thromboembolisms (i.e. pulmonary embolism or deep-vein thrombosis) and peripheral arterial thrombosis.

This invention relates, more particularly, to the use or utilization of MMP-10 and a plasminogen activator in the preparation of a medicament or pharmaceutical composition for thrombolytic therapy and treatment by means of simultaneous, separate or sequential administration.

Within the context of this invention, a plasminogen activator is a compound which activates the conversion of inactive plasminogen into plasmin by means of the cleavage of the peptide bond between Arg560 and Val1561 of the plasminogen. In particular, these plasminogen activators include: urokinase (uPA), tissue plasminogen activator (tPA), streptokinase and staphylokinase.

In one particular embodiment of this invention, the plasminogen activator is tPA.

In another particular embodiment, the plasminogen activator is uPA.

In yet another particular embodiment, a derivative or fragment of the aforementioned plasminogen activators can be used as the plasminogen activator which retains its ability to cleave and activate the plasminogen for which the effect of facilitating the MMP-10 is effective. An expert in the field can readily see this effect by himself, for example by means of *in vitro* clot formation and lysis tests such as those described in examples 1 to 4 of this invention. Longstaff and Thelwell [FEBS Letters, 2005; 579: 3303-3309] review some of the plasminogen activators currently in use or in development, from among which an expert can choose, for using them in combination with MMP-10 according to the present invention.

Advantageously, on not interacting with the circulating fibrinogen/fibrin and being capable of facilitating the action of the plasminogen activators, MMP-10 would make it possible to reduce the dose of the thrombolytic by maintaining the effectiveness for lysing the clot, but without inducing systemic fibrinolysis, which would entail a great incidence of hemorrhaging-related complications. Similarly, it would make it possible to reduce the toxicity resulting from the thrombolytic treatment with agents such as tPA.

According to another aspect, the present invention also relates to a pharmaceutical combination which comprises, separately, or in one same composition, MMP-10 and a plasminogen activator, mixed with pharmaceutically-acceptable excipients or vehicles. The plasminogen activator in the combination may be any of those mentioned hereinabove.

The aforesaid combination is useful for thrombolytic therapy and treatment in mammals, particularly in humans, in any of the aforementioned clinical conditions stated hereinabove.

The origins of MMP-10 and of the plasminogen activator in the pharmaceutical combination are not a critical aspect of this invention. The active ingredients may be obtained by extraction and purification from biological fluids or tissues by means of recombinant or genetic engineering procedures or any other conventional technique.

Depending on the circumstances, to be determined in each case by means of the customary pharmacological and clinical tests, the active ingredients of the pharmaceutical combination can be administered simultaneously, separately or sequentially.

According to one embodiment of the invention, the active ingredients (MMP-10 and plasminogen activator) can be contained in one some pharmaceutical composition. In other cases, the active ingredients can be contained in separate pharmaceutical compositions, each one thereof in its own container mixed with pharmaceutically-acceptable excipients or vehicles.

The pharmaceutical compositions with the active ingredients whether one or more, can be formulated in both solid form (i.e. freeze-dried in vials to later be reconstituted in a suitable solution) or also in liquid form.

In one particular embodiment, these compositions with the active ingredients constitute a kit for thrombolytic therapy or treatment which may optionally include other components, such as: containers with solutions for reconstituting the active ingredients, cannulas, drip bags with physiological serum for intravenous application and instructions for use, etc.

The pharmaceutical compositions with the active ingredients may be administered by any suitable route, for example, orally, parenterally, rectally or topically, for which they will include the pharmaceutically-acceptable excipients and vehicles necessary for the formulation of the desired form of administration.

In one particular embodiment, administration is parenteral, for example by intravenous injection, or administered locally by catheterization for *in situ* administration in the near vicinity of the clot.

When the pharmaceutical combination is for administering separately, both active ingredients may also be contained in pharmaceutical compositions suitable for administering by different routes.

The quantities of MMP-10 and of plasminogen activator which may be present in the compositions of the pharmaceutical combination provided by this invention may vary within a broad range, but always in therapeutically effective quantities.

The dosage for each thrombolytic treatment protocol with the compositions of the pharmaceutical combination of this invention will depend on numerous factors, including the patient's age, condition, the severity of the clinical condition to be treated, the route and frequency of administration and of the plasminogen activator which is going to be administered in each case.

In one typical embodiment, the quantities and doses of the plasminogen activator will be smaller than those which would be used for this same plasminogen activator when MMP-10 is not included in the therapeutic combination. On the other hand, the quantities of MMP-10 will be adjusted in terms of the effect one wishes to achieve: greater thrombolytic effectiveness by maintaining the plasminogen activator dosage; or a reduction of the plasminogen activator dose maintaining the thrombolytic effectiveness.

In another aspect, this invention relates to a pharmaceutical combination or a kit of the invention, as have already been described, for thrombolytic therapy.

In another additional aspect, the invention also relates to a method for thrombolytic treatment and therapy comprising the administration to the patient of a therapeutically effective quantity of MMP-10. In one particular embodiment, said method also comprises the administration of a plasminogen activator, by simultaneous, separate or sequential administration. Any of the aforementioned pharmaceutical compositions and combinations could be used for this method.

The following examples illustrate this invention and must not be considered limiting of the scope thereof.

### EXAMPLES OF THE INVENTION

The examples illustrate the effects on the fibrinolytic and thrombolytic activity of the MMP-10 and MMP-3 matrix metalloproteinases, either directly or in combination with some plasminogen activators: urokinase (uPA) and tissue plasminogen activator (tPA).

The following has been employed for the examples:
- Recombinant MMP-10, obtained as 58 kDa enzyme with 20%-30% mature 48 kDa enzyme (R&D Systems, 910-MP, Abingdon, UK), which was reconstituted with TCNB buffer (50 mM Tris-HCl, pH 7.5, 10 nM CaCl₂, 150mM NaCl, 0.05 % Brij35).
- Recombinant MMP-3, obtained as 52 kDa enzyme (R&D Systems, 513-MP, Abingdon, UK), supplied in a solution with 12.5 nM Tris, 5 nM CaCl₂, 0.025% Brij35 and 50% glycerol.
- Urokinase (uPA) (Vedim Pharma SA; 628602, Barcelona, Spain).
- Recombinant plasminogen tissue activator (tPA) (Boerhinger Ingelheim; 985937 Actilyse®, Ingelheim, Germany).

For the evaluation of the thrombolytic activity, a turbidimetric method was used for monitoring the formation and lysis of the fibrin clot on plasma samples according to the protocol previously described by von dern Borne et al., [Blood, 1995; 86:3035-3042].

On the other hand, for evaluating the activity on the fibrin lysis, tests were conducted on fibrin plates following the procedure described by Edward [J. Clin. Path., 1972: 25: 335-337].

### Example 1: Effect of MMP-10 and MMP-3 on clot formation and lysis

As previously mentioned hereinabove, by means of the procedure described by von darn Borne et al., an evaluation was conducted of the effect of MMP-10 and MMP-3 on the haemostatic system. In this method, the changes in turbidity/absorbancy during the formation and lysis of clots are assessed as an indicator of the length of both of these processes. The turbidity is measured by reading the absorbance at 405 nm during the clot formation and lysis phases using a photometric reader, which, in our case, was an ELISA reader (Fluostar Optima, BMG Labtech). The increase in turbidity/absorbancy indicates the formation of the fibrin clot, whilst the lessening of this parameter indicates the lysis of the clot.

For the formation of the clot, 75 µl citrated plasma, 75 µl HEPES buffer (25 mM HEPES, 137 mM NaCl, 3.5 mM KCI, 6 mM CaCl₂, 1.2 mM Mg Cl₂, and 0.1 % BSA, pH=7.5) and 10 µl CaCl₂ 150 mM were mixed in a microplate well. The plate was incubated to 37°C and the absorbance at 405 nM measured for 2 hours every 30 seconds.

To study the effect of the MMP-10 on clot formation, activated MMP-10 (50, 100 and 200 nM) was added to the initial plasma and HEPES buffer mixture. Prior to its use in the experiments, the MMP-10 was activated by means of heat treatment at 37°C for 1 hour.

In parallel tests, the effect on clot formation was also analyzed with the MMP-3 (200 nM). In this case, the MMP-3 was previously activated with 1 mM p-aminophenylmercury acetate (APMA, 164610, EMD Biosciences, La Jolla, USA) at 37°C for 24 h.

As is shown in Fig. 1A, the MMP-10 did not induce any changes in either the speed of the clot formation or in the maximum degree of turbidity reached with any of the doses employed (Table 1). However, the MMP-3 induced a 50% drop in the maximum absorbancy/turbidity of the clot formed, probably due to it direct proteolytic action on the fibrinogen.

These results show that the MMP-10, unlike what was described for the MMP-3, does not alter the clot-formation rate on not displaying any fibrinolytic activity regarding the fibrinogen.

Afterwards, a study was conducted of the fibrin clot lysis rate and, to this end, as in the immediately preceding section hereinabove, recalcified plasma in HEPES buffer was used, to which, simultaneously with the MMP-10 (or MMP-3, as the case may be), a plasminogen activator was added in order to select either 30 U/ml of tissue plasminogen activator (tPA) or 135 U/ml urokinase (uPA) at the beginning of the turbidimetry.

The concentrations of tPA and uPA to be used were determined in prior dose-response studies, where the dose of choice was that which completely lyses the fibrin clot within a two-hour (2h) time period.

As is shown in Fig. 1B and Table 1, the MMP-10 in absence of tPA and uPA did not cause the lysis of the fibrin clot, whilst in the presence of the tPA or uPA activators, it induced a significant increase in the fibrin clot lysis rate. With the maximum dose of MMP-10 tested (200 nM), the shortening of the lysis time (time at which half of the clot lyses) was 15 min (52.9 min vs. 68.3 min, p<0.01) in the presence of tPA and 5 min in the presence of uPA (42 min vs. 47.5 min, p<0.05). This reduction in the lysis time means a 20% shortening in the presence of tPA and a 10% shortening with the uPA.

To the contrary, the MMP-3 did not change the clot lysis rate in the presence of tPA.

These findings indicate that MMP-10, unlike MMP-3, is not capable of digesting the fibrin but does heighten the fibrinolytic effect of the plasminogen activators and of the fibrinolysis (tPA or uPA). On not having the ability to act on the endogenous fibrinolysis, the MMP-10 would prevent or attenuate the onset of hemorrhages, which makes it a good candidate for being used as a coadjuvant of thrombolytic therapy.

**Table 1. Fibrin clot lysis time (given in minutes), in the presence of plasminogen activators (tPA or uPA).**

| | TPA 30 | tPA 20 | tPA 15 | uPA 135 |
|---|---|---|---|---|
| | U/ml | U/ml | U/ml | U/ml |
| Control | 68.3 | 102.0 | 125.3 | 47.5 |
| MMP-10 50 nM | 65.5 | - | - | - |
| MMP-10 100 nM | 61.2 | - | - | - |
| MMP-10 200 nM | 52.9 | 84.7 | 108.7 | 42.0 |
| MMP-3 200 nM | 76.3 | - | - | - |
| Anti-MMP-10 (MAb) | No lysis | - | - | No lysis |
| (IgG) Isotype control | 74.3 | - | - | 48.3 |

### Example 2: Effect of MMP-10 on fibrin degradation

According to the aforementioned Edward procedures, a study was made of the effect on fibrin lysis by measuring the halo or lysis area which is caused on a polymerized fibrin plate.

The fibrin plates are prepared from a 6 mg/ml human fibrinogen solution (Sigma, F3879, Saint Louis, MO, USA) in veronal buffer (BioWhittaker, 12-624E, Cambrex, MD, USA) at 37°C, which is filtered and to which an equal volume of CaCl₂ (50 mM) is added. This solution (6 ml) is mixed with 1 international unit (NIH units) thrombin (Enzyme Research Lab; HT1200a, Swansea, UK) and is left to polymerize for 6 h.

To assess the fibrinolytic capacity, tPA (1 U/ml), MMP-10 (200 nM) or a combination of the two were added to different fibrin plates.

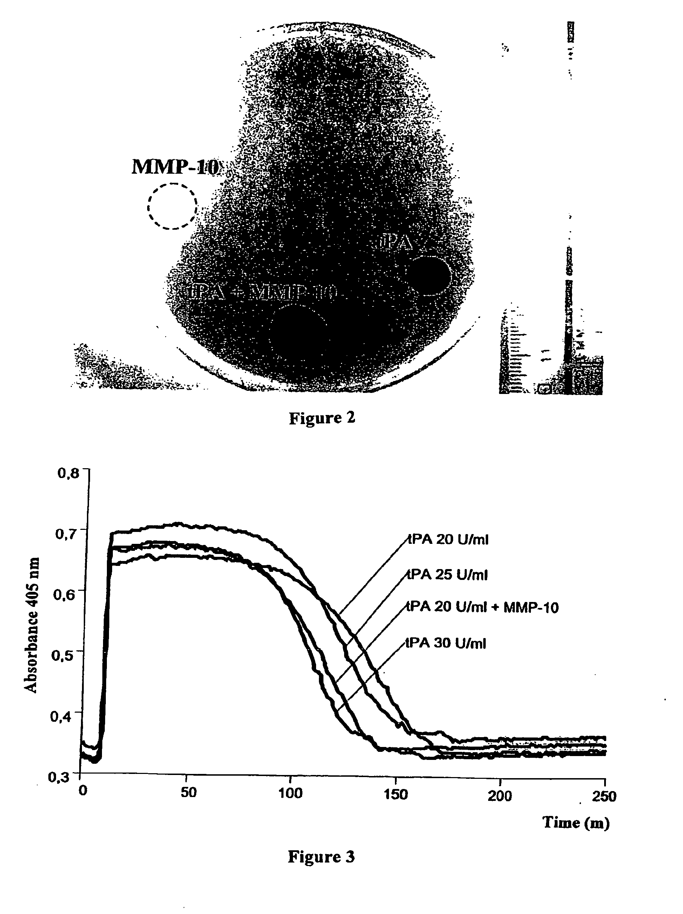

As is shown in Fig. 2, the MMP-10 alone did not cause lysis on the polymerized fibrin, whilst the tPA caused a marked halo. However, the combination of tPA and MMP-10 significantly increased the polymerized fibrin lysis area (188.6%), this being a fact which confirms the fibrinolysis-facilitating effect that MMP-10 has in combination with plasminogen activators as fibrinolytic agents.

### Example 3: Effect of coadministration of MMP-10 and a thrombolytic agent (tPA or uPA) on clot lysis

Given the effect of the MMP-10 on the tPA-included lysis, the question was posed of ascertaining whether it is possible to reduce the dose of tPA (which entails hemorrhage and neurological toxicity-related problems) and use MMP-10 as a coadjuvant for achieving the same thrombolytic effect.

In the turbidimetry test conducted following the procedure described in Example 1 hereinabove, we found that the presence of MMP-10 (200 nM) in combination with the tPA makes it possible to reduce the dose of tPA by 33% (from 30 to 20 U/ml), achieving the same clot lysis time (Fig. 3, table 1).

This result indicates that in a subject who needs thrombolytic therapy, MMP-10 provides the way to increase the fibrinolysis and clot lysis by simultaneously lowering the dose of tPA and therefore, minimizing the hemorrhagic and toxicity-related problems caused by this drug.

### Example 4: Inhibition of clot fibrinolysis and lysis induced by tPA with anti-MMP-10 antibodies

According to the results of Examples 1 and 2, an analysis was made of the specificity of the effect of the MMP-10 on fibrin lysis in the clot induced by tPA by simultaneously adding different doses of active MMP-10 in the presence (1:2 ratio) and absence of a monoclonal antibody which blocks its activity (R&D systems, MAB9101, Abingdon, UK), or of lgG2B murine isotype control antibody (eBioscience, 16-4732, San Diego, CA, USA) at the same concentration as the antibody.

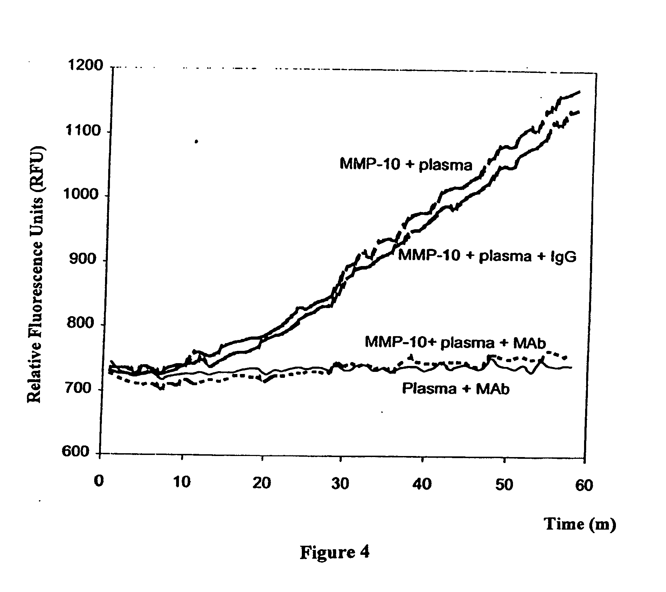

The enzyme:antibody ratio which blocks the activity of the enzyme was previously analyzed in an activity test for the MMP-10 on a microdish covered with an anti-MMP-10 antibody (R&D Systems, Clon110343) and using the fluorogenic stromelysin substrate (MCA-Arg-Pro-Lys-Pro-Val-Glu-Nval-Trp-Arg-Lys-[DNP]-NH₂) (R&D Systems; ES002, Abingdon, UK) [Lombard et al.; Biochimie, 2005; 87:265-272). The fluorescence (320 nm excitation and 405 nM emission) was measured on a spectrofluorimeter (SpectraMAX GeminiXS, Molecular Devices, CA, USA) for 1 h, it having been found that the 1:2 ratio completely inhibits the concentration of active enzyme (Fig. 4).

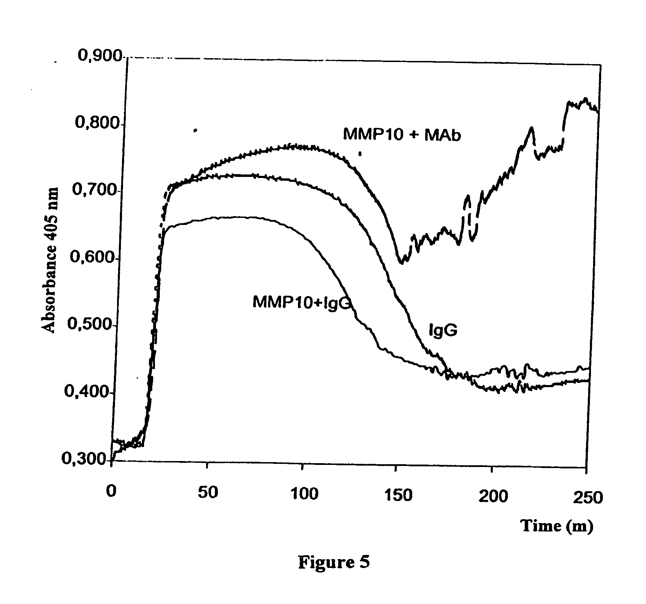

The results show the coadjuvant effect on the fibrinolysis to be MMP-10 specific, given that it reverses in the presence of the anti-MMP-10 antibody. This effect is quite remarkable when said antibody is added to block the endogenous activity of the plasma MMP-10 (Fig. 5). The results reveal that the absence of MMP-10 in the plasma prevents the lysis of the fibrin clot even in the presence of tPA or uPA (Table 1).

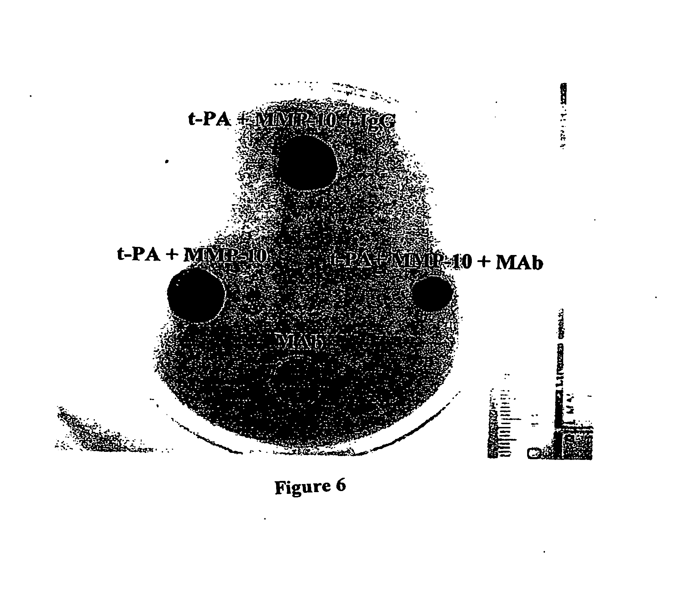

These results were corroborated in the polymerized fibrin plate tests. As is shown in Fig. 6, in the presence of the anti-MMP-10 antibody, the lysis area caused by the tPA: MMP-10 combination is reduced (91.2% vs. 188:6%), whilst the control antibody has no effect (184.6%). This data confirm that employing an MMP-10 inhibitor antibody blocks the pharmacological dissolving of fibrin clots.

## Claims

1. Use of matrix metalloproteinase-10 MMP-10 in the preparation of a medicament for thrombolytic therapy.

2. Use according to claim 1, **characterized in that** said medicament also comprises a plasminogen activator selected from among: the tPA tissue plasminogen activator, uPA urokinase activator, streptokinase, staphylokinase and a derivative or fragment of said plasminogen activators which retains its ability to activate the plasminogen.

3. Use according to claim 2, **characterized in that** the medicament is suitable for simultaneous, separate or sequential administration of said components.

4. Use according to claim 2, **characterized in that** the plasminogen activator is selected from among: tPA and a derivative or fragment of tPA which retains its ability to activate the plasminogen.

5. Use according to claim 4, **characterized in that** the plasminogen activator is tPA.

6. Use according to claim 2, **characterized in that** the plasminogen activator is selected from among: uPA and a derivative or fragment of uPA which retains its ability to activate the plasminogen.

7. Use according to claim 6, **characterized in that** the plasminogen activator is uPA.

8. A pharmaceutical combination for simultaneous, separate or sequential administration, **characterized in that** it comprises a matrix metalloproteinase-10 MMP-10 and a plasminogen activator mixed with pharmaceutically-acceptable excipients or vehicles, wherein the plasminogen activator is selected from among: the tPA tissue plasminogen activator, uPA urokinase activator, streptokinase, staphylokinase and a derivative or fragment of said plasminogen activators which retains its ability to activate the plasminogen.

9. A pharmaceutical combination according to claim 8, **characterized in that** the plasminogen activator is tPA.

10. A pharmaceutical combination according to claim 8, **characterized in that** the plasminogen activator is uPA.

11. A kit **characterized in that** it comprises the matrix metalloproteinase-10 MMP-10 and a plasminogen activator as a pharmaceutical combination for their simultaneous, separate or sequential administration for thrombolytic treatment, wherein the plasminogen activator is selected from among: the tPA tissue plasminogen activator, uPA urokinase activator, streptokinase, staphylokinase and a derivate or fragment of said plasminogen activators which retains its ability to activate the plasminogen.

12. A kit according to claim 11, **characterized in that** the plasminogen activator is tPA.

13. A kit according to claim 11, **characterized in that** the plasminogen activator is uPA.

14. Matrix metalloproteinase-10 MMP-10 for use in thrombolytic therapy.

15. A pharmaceutical combination according to any of claims 8-10 or a kit according to any of claims 11-13 for thrombolytic therapy.

## Patentansprüche

1. Verwendung einer Matrix-Metalloproteinase-10 (MMP-10) in der Herstellung eines Medikaments für eine thrombolytische Therapie.

2. Verwendung gemäß Anspruch 1, **dadurch gekennzeichnet, dass** das Medikament auch einen Plasminogenaktivator umfasst, ausgewählt aus: dem tPA-Gewebeplasminogenaktivator, uPA-Urokinaseaktivator, Streptokinase, Staphylokinase und einem Derivat oder Fragment der Plasminogenaktivatoren, welches seine Fähigkeit zur Aktivierung des Plasminogens beibehält.

3. Verwendung gemäß Anspruch 2, **dadurch gekennzeichnet, dass** das Medikament zur gleichzeitigen, separaten oder aufeinanderfolgenden Verabreichung der Komponenten geeignet ist.

4. Verwendung gemäß Anspruch 2, **dadurch gekennzeichnet, dass** der Plasminogenaktivator ausgewählt ist aus: tPA und einem Derivat oder Fragment von tPA, welches seine Fähigkeit zur Aktivierung des Plasminogens beibehält.

5. Verwendung gemäß Anspruch 4, **dadurch gekennzeichnet, dass** der Plasminogenaktivator tPA ist.

6. Verwendung gemäß Anspruch 2, **dadurch gekennzeichnet, dass** der Plasminogenaktivator ausgewählt ist aus: uPA und einem Derivat oder Fragment von uPA, welches seine Fähigkeit zur Aktivierung des Plasminogens beibehält.

7. Verwendung gemäß Anspruch 6, **dadurch gekennzeichnet, dass** der Plasminogenaktivator uPA ist.

8. Pharmazeutische Kombination zur gleichzeitigen, separaten oder aufeinanderfolgenden Verabreichung, **dadurch gekennzeichnet, dass** sie eine Matrix-Metalloproteinase-10 (MMP-10) und einen Plasminogenaktivator in Mischung mit pharmazeutisch akzeptablen Exzipienten oder Vehikeln umfasst, wobei der Plasminogenaktivator ausgewählt ist aus: dem tPA-Gewebeplasminogenaktivator, uPA-Urokinaseaktivator, Streptokinase, Staphylokinase und einem Derivat oder Fragment der Plasminogenaktivatoren, welches seine Fähigkeit zur Aktivierung des Plasminogens beibehält.

9. Pharmazeutische Kombination gemäß Anspruch 8, **dadurch gekennzeichnet, dass** der Plasminogenaktivator tPA ist.

10. Pharmazeutische Kombination gemäß Anspruch 8, **dadurch gekennzeichnet, dass** der Plasminogenaktivator uPA ist.

11. Kit, **dadurch gekennzeichnet, dass** er die Matrix-Metalloproteinase-10 (MMP-10) und einen Plasminogenaktivator als einer pharmazeutischen Kombination für ihre gleichzeitige, separate oder aufeinanderfolgende Verabreichung für eine thrombolytische Behandlung umfasst, wobei der Plasminogenaktivator ausgewählt ist aus: dem tPA-Gewebeplasminogenaktivator, uPA-Urokinaseaktivator, Streptokinase, Staphylokinase und einem Derivat oder Fragment der Plasminogenaktivatoren, welches seine Fähigkeit zur Aktivierung des Plasminogens beibehält.

12. Kit gemäß Anspruch 11, **dadurch gekennzeichnet, dass** der Plasminogenaktivator tPA ist.

13. Kit gemäß Anspruch 11, **dadurch gekennzeichnet, dass** der Plasminogenaktivator uPA ist.

14. Matrix-Metalloproteinase-10 (MMP-10) zur Verwendung in der Thrombolysetherapie.

15. Pharmazeutische Kombination gemäß jedem der Ansprüche 8-10 oder Kit gemäß jedem der Ansprüche 11-13 für eine thrombolytische Therapie.

## Revendications

1. Utilisation de la métalloprotéinase-10 matricielle MMP-10 dans la préparation d'un médicament pour la thérapie thrombolytique.

2. Utilisation selon la revendication 1, **caractérisée en ce que** le médicament comprend également un activateur de plasminogène choisi parmi l'activateur de plasminogène tissulaire tPA, l'activateur de type urokinase uPA, la streptokinase, la staphylokinase et un dérivé ou fragment desdits activateurs de plasminogène, qui conserve l'aptitude à activer le plasminogène.

3. Utilisation selon la revendication 2, **caractérisée en ce que** le médicament est approprié pour une administration simultanée, séparée ou séquentielle desdits composants.

4. Utilisation selon la revendication 2, **caractérisée en ce que** l'activateur de plasminogène est choisi parmi le tPA et un dérivé ou fragment du tPA, qui conserve son aptitude à activer le plasminogène.

5. Utilisation selon la revendication 4, **caractérisée en ce que** l'activateur de plasminogène est le tPA.

6. Utilisation selon la revendication 2, **caractérisée en ce que** l'activateur de plasminogène est choisi parmi l'uPA et un dérivé ou fragment de l'uPA, qui conserve son aptitude à activer le plasminogène.

7. Utilisation selon la revendication 6, **caractérisée en ce que** l'activateur de plasminogène est l'uPA.

8. Combinaison pharmaceutique pour administration simultanée, séparée ou séquentielle, **caractérisée en ce qu'**elle comprend une métalloprotéinase-10 matricielle MMP-10 et un activateur de plasminogène, mélangés à des excipients ou véhicules pharmaceutiquement acceptables, où l'activateur de plasminogène est choisi parmi l'activateur de plasminogène tissulaire tPA, l'activateur de type urokinase uPA, la streptokinase, la staphylokinase et un dérivé ou fragment desdits activateurs de plasminogène, qui conserve l'aptitude à activer le plasminogène.

9. Combinaison pharmaceutique selon la revendication 8, **caractérisée en ce que** l'activateur de plasminogène est le tPA.

10. Combinaison pharmaceutique selon la revendication 8, **caractérisée en ce que** l'activateur de plasminogène est l'uPA.

11. Kit **caractérisé en ce qu'**il comprend la métalloprotéinase-10 matricielle MMP-10 et un activateur de plasminogène, sous forme d'une combinaison pharmaceutique pour leur administration simultanée, séparée ou séquentielle pour le traitement thrombolytique, où l'activateur de plasminogène est choisi parmi l'activateur de plasminogène tissulaire tPA, l'activateur de type urokinase uPA, la streptokinase, la staphylokinase et un dérivé ou fragment desdits activateurs de plasminogène, qui conserve l'aptitude à activer le plasminogène.

12. Kit selon la revendication 11, **caractérisé en ce que** l'activateur de plasminogène est le tPA.

13. Kit selon la revendication 11, **caractérisé en ce que** l'activateur de plasminogène est l'uPA.

14. Métalloprotéinase-10 matricielle MMP-10 à utiliser en thérapie thrombolytique.

15. Combinaison pharmaceutique selon l'une quelconque des revendications 8 à 10 ou kit selon l'une quelconque des revendications 11 à 13 pour la thérapie thrombolytique.
